(19) Europäisches Patentamt / European Patent Office / Office européen des brevets

(11) **EP 3 554 479 B1**

(12) **EUROPEAN PATENT SPECIFICATION**

(45) Date of publication and mention
of the grant of the patent:
**16.02.2022 Bulletin 2022/07**

(21) Application number: **17829184.5**

(22) Date of filing: **19.12.2017**

(51) International Patent Classification (IPC):
**A23P 10/20** (2016.01)      **A61K 9/16** (2006.01)
**A61K 9/50** (2006.01)

(52) Cooperative Patent Classification (CPC):
**A61K 9/1658; A61K 9/1652; A61K 9/5036;
A61K 9/5052**

(86) International application number:
**PCT/EP2017/083583**

(87) International publication number:
**WO 2018/114971 (28.06.2018 Gazette 2018/26)**

(54) **A METHOD FOR PRODUCING BEADS**

VERFAHREN ZUR HERSTELLUNG VON KÜGELCHEN

PROCÉDÉ DE PRODUCTION DE BILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2016 EP 16205148**

(43) Date of publication of application:
**23.10.2019 Bulletin 2019/43**

(73) Proprietor: **Agriculture and Food Development
Authority
(TEAGASC)
Co. Carlow (IE)**

(72) Inventors:
• **BRODKORB, Andre
Fermoy
Co. Cork (IE)**
• **HAQUE, Kamrul
Fermoy
Co. Cork (IE)**

(74) Representative: **Purdylucey Intellectual Property
6-7 Harcourt Terrace
D02 FH73 Dublin 2 (IE)**

(56) References cited:
**WO-A1-02/094224**

• HEIDEBACH T ET AL: "Microencapsulation of probiotic cells by means of rennet-gelation of milk proteins", FOOD HYDROCOLLOIDS, ELSEVIER BV, NL, vol. 23, no. 7, 1 October 2009 (2009-10-01), pages 1670-1677, XP026145859, ISSN: 0268-005X, DOI: 10.1016/J.FOODHYD.2009.01.006 [retrieved on 2009-01-19]
• TAVARES GUILHERME M ET AL: "Milk proteins as encapsulation devices and delivery vehicles: Applications and trends", TRENDS IN FOOD SCIENCE AND TECHNOLOGY, vol. 37, no. 1, 1 January 2014 (2014-01-01), pages 5-20, XP028655072, ISSN: 0924-2244, DOI: 10.1016/J.TIFS.2014.02.008
• LUCIE BEAULIEU E A: "Elaboration and Characterization of Whey Protein Beads by an Emulsification/Cold Gelation Process: Application for the Protection of Retinol", MACROMOLECULES, AMERICAN CHEMICAL SOCIETY, US, vol. 3, no. 2, 22 January 2002 (2002-01-22), pages 239-248, XP002515664, ISSN: 0024-9297, DOI: 10.1021/BM010082Z [retrieved on 2002-01-22]
• CHEN L ET AL: "Alginate-whey protein granular microspheres as oral delivery vehicles for bioactive compounds", BIOMATERIALS, ELSEVIER SCIENCE PUBLISHERS BV., BARKING, GB, vol. 27, no. 26, 1 September 2006 (2006-09-01), pages 4646-4654, XP027951264, ISSN: 0142-9612 [retrieved on 2006-09-01]

**Description**

**Field of the Invention**

The current invention relates to a method for producing beads. In particular, the current invention relates to a method for producing microbeads. The invention also relates to a microbead preparation produced by the method of the invention.

**Background of the Invention**

Microbeads are spherical polymer particles, typically from about 1 μm to 1000 μm in diameter. Typically, spherical polymer particles, larger than 1mm in diameter are called macrobeads. Beads, particularly microbeads, are widely used as vehicles to protect, transport and control the release of a bioactive component(s), such as flavours, vitamins, peptides, enzymes, antibodies and microorganisms, encapsulated therein, to a target site in the body in both the food and pharmaceutical fields. Microbead preparations are often administered by oral ingestion via a food or beverage product.

Generally speaking, microbead preparations are made using a method comprising a first step of producing a polymer droplet. This step is followed by gelation of the droplet by changing the solvent properties or the environment of the polymer mixture (e.g. variation in temperature, pH, ionic strength).

Biopolymers, especially those representing as non-toxic, low cost, biocompatible and biodegradable are frequently used as effective encapsulating matrix components in food and pharmaceutical applications. As crosslinkers are commonly used to form the polymer microbeads, the polymers chosen to prepare the microbeads are generally those which react well with a crosslinker. Alginate, or alginic acid, is one of the most widely used biopolymers in the manufacture of microbeads due to its simplicity, non-toxicity, biocompatibility and gelling properties. Alginate is a linear copolymer with homopolymeric blocks of linked (1-4)-linked β-D-mannuronate and α-L-guluronate residues and it is capable of crosslinking with calcium ions to form microbeads. However, alginate microbeads have some drawbacks in that they offer limited protection of active agents, such as probiotic bacteria, when ingested due to their low stability in the acidic conditions of the stomach.

In an attempt to combat this problem, microbeads have been developed previously, which comprise a combination of alginate and heat denatured whey protein. These microbeads are produced by crosslinking this mixture with calcium ions. Whey proteins are biopolymers that are used in the food industry for their nutritional value and functional ability to form gels and emulsions. However, the whey protein isolate (WPI) used in the manufacture of these microbeads is relatively expensive and therefore, not suitable for large scale commercial production. WPI also requires a heat denaturation step to activate its gelation properties. This step increases solution viscosity significantly, which can cause problems during production as it is difficult to filter (Doherty, et al.). Milk protein-based micro-beads have been used for successful encapsulation of bioactives including probiotic bacteria (Hébrard, et al., 2009; Doherty et al., 2011; Wichchukit et al., 2013; Shi et al., 2013; Egan et al., 2014; O'Neill et al., 2014).

There are several methods used for production of microbeads using alginate, pectin, denatured whey protein or other polymers, known in the field. However, each of these methods has certain limitations. For example, Ainsley *et al.,* describes a method of production of droplets using syringe or needle pump system and subsequent gelation of the droplet having a size of about 1mm to 3mm in a gelling bath. As this method produces microbeads with a relatively large size, it is not suitable for production of microbeads having food applications due to the adverse mouth feel of the beads. Other methods used in the field involve the emulsification (oil in water) of denatured whey protein by high pressure homogenisation or high shear, followed by internal calcium mediated gelation and subsequent separation of gel beads from oil phase. In contrast to the former method, this method produces beads of a very small size, (<100). These microbeads would be suitable for use and easily incorporated in a variety of food systems. However, high pressure and high sheer used in this method may not be feasible for encapsulation and protection of active agents such as probiotic bacteria. A further method described by Doherty *et al.,* involves extrusion of a polymer mixture such as alginate and denatured whey protein from a syringe through a vibrating nozzle to generate uniform droplets under the effects of vibration, followed by the subsequent gelation of the droplets in a gel bath. This method produces microbeads of intermediate size (between 150 and 900um in diameter depending on the nozzle size used), which are also suitable for food application. However, as stated above, there are several drawbacks to using alginate and denatured whey protein in this method. In particular, the increased solution viscosity imparted by the denaturation step of the WPI hampers the filtration step of the extrusion technique.

Folate is a water-soluble B vitamin that is naturally present in some foods. Naturally occurring folates in reduced form are highly sensitive to oxygen, temperature, pH and light and thus their stability is affected during processing and storage of food sources of this vitamin. Folic acid refers to the synthetic, fully oxidise form of folate that is used in dietary supplements and fortified foods. Folic acid is relatively stable between pH 5 and 12 in aqueous solution when protected from light, even when it is heated. However, as the pH decreases below 5, it is susceptible to aerobic hydrolysis to give p-aminobenzoylglutamic acid and 6-methylpterin.

WO02/094224 discloses a method for producing a bead in which chitosan modified with caproic acid was mixed with lactic bacteria in the presence of milk proteins. The beads were then formed and subsequently re-suspended in alginate. No heat-treated milk protein was used and the beads of this document were formed before the addition of alginate. In view of the use of chitosan and the fact that both alginate and chitosan were modified by succinylation, these beads would not be suitable for use in the food industry.

This document discloses a further method for producing a bead using a solution of alginate mixed with a solution of whey protein and lactic bacteria. The suspension was dripped into a further solution of alginate and the beads were formed. Again, this method does not use heat-treated proteins. Using whey protein without heat-treatment provides beads which are structure by calcium alginate. In other words, the protein did not aid in the bead formation but acted as a filler. As a result, the beads of WO02/094224 are weak and lack optimum stability.

The current invention aims to alleviate one or more of the above problems by providing a method for producing a preparation of beads, preferably microbeads, which is low cost, less energy intensive, suitable for incorporation of sensitive bioactive components and permits large scale production. The beads produced by the method of the invention are robust have a high nutritional value and are suitable for food and/or beverage applications.

## Summary of the Invention

The current invention provides a method for producing beads comprising an active component encased therein, the method comprising

- providing a solution comprising a milk protein product in which the milk protein has at least a 70% degree of denaturation, alginate and an active component
- forming a droplet with said solution, and
- gelation of the droplet in a bath of a solution comprising a cation, to form a bead(s).

Preferably, the milk protein product or milk protein is heat-treated.

Preferably, the beads are microbeads.

Suitably, the beads are macrobeads.

Preferably, the milk protein product is milk protein isolate (MPI). Alternatively, the milk protein product is milk protein concentrate (MPC).

Preferably, alginate is sodium alginate.

Preferably, the concentration of the milk protein product in the solution is from about 0.5% w/w to about 6% w/w. Typically, said concentration is from about 1.5% w/w to about 3% w/w. Still preferred, said concentration is 2% w/w. Preferably, the concentration is less than about 5%. Typically, the concentration is less than about 3% w/w.

Preferably, the concentration of the alginate in the solution is from about 0.3% to about 3% w/w. Typically, said concentration is from about 0.5% to about 0.8% w/w. Still preferred, said concentration is about 0.7% w/w.

Preferably, the concentration of the active component in the solution is greater than 0 to about 4%w/w. Typically, said concentration is from about 0.004%w/w to about 0.4%w/w. Ideally, said concentration is about 0.04%w/w.

Preferably, the MPI comprises at least about 80% milk protein.

Typically, the MPC comprises between about 35% and about 80% milk protein. Preferably, about 65% milk protein.

Typically, the heat-treated milk protein product, or milk protein, has a degree of denaturation of at least 80%.

Typically, gelation occurs immediately after the formation of droplets.

Preferably, said cation is selected from the group comprising calcium, magnesium and iron.

Preferably, said cation is calcium ions.

Suitably, the cation is calcium chloride,

Typically, the bath contains a solution comprising about 0.1 M to about 0.5M of cation. Preferably, from about 0.1M to about 0.3M. Ideally, about 0.2M.

Preferably, an effective amount of Tween-20 is added to the bath.

Preferably, the solution is stirred during gelation.

Preferably, the droplet is produced by extrusion. Typically, this method comprises extrusion of the mixture from a syringe through a vibrating nozzle.

Alternatively, the droplet is produced by jet-cutting.

Typically, said spray nozzle has an aperture of from about 100 μm to about 1mm. Preferably, said aperture is from about 150 μm, to about 400 μm. Still preferred, said aperture is ideally 200 μm.

Alternatively, the droplet is produced using a nozzle having an aperture of from 0.5mm to about 2.5mm, preferably about 1mm or larger. The droplet may be produced using a pipette, a burette, or a syringe, having an aperture of from 0.5mm to about 2.5mm, preferably about 1mm or larger.

Typically, gelation is for a period of about 15 mins to 60 mins. Preferably, gelation is with agitation.

Typically, the frequency operation of the vibrating nozzle is from about 1000 Hz to 4000 Hz. Generally, the frequency is from about 1200Hz to about 2000Hz. Still preferred, the frequency is from about 1500Hz to about 1800 Hz.

Typically, the flow rate of the mixture through the nozzle is from about 4.6 to 5.8 ml/min. Preferably, the flow rate is from about 3.5 to about 6.5 ml/min. Still preferred, the flow rate is from about 4.6 to 6.0 ml/min. Preferred, the flow rate is about 5.7 ml/min.

Preferably, the falling distance from the nozzle to the bath is from about 8 cm to about 25 cm. Preferably, the falling distance is from about 10 cm to about 18 cm. Ideally, the falling distance is about 14cm.

Preferably, the electrostatic potential between the nozzle and bath is from about 0.8 to 2kV. Preferably, the electrostatic potential is from about 0.95kV to about 1.15kV. Preferably, the electrostatic potential is about 1kV.

Preferably, the amplitude range is from about 1 to about 7. Preferably, from about 3 to about 7. Ideally, the amplitude range is about 6.

Preferably, the mixture is filtered prior to the steps of forming the droplets and gelation.

The size of the gel bath is such that it is large enough to ensure enough cations and space are available for bead formation.

Preferably, the active component is selected from the group comprising a probiotic, a prebiotic, an antibody, an enzyme, a vitamin, preferably a light sensitive vitamin, a microorganism, a protein, a sugar, a nucleic acid or nucleic acid construct and a pharmaceutically active agent, or a combination thereof.

Typically, said vitamin is folic acid. Alternatively, the active component is beta-carotene.

Suitably, the active component is homogeneously dispersed within the microbead or macrobead.

Typically, the microbead or macrobead produced is spherical.

Typically, the microbead or microbead preparation is uniform in shape.

Preferably, the microbeads in the preparation of microbeads have a size range of about about 1 to about 1,000$\mu$m, in other words, less than or equal to 1mm. Preferably, the microbeads in the preparation have a size range from about 300 to about 450 $\mu$m.

Preferably, the macrobeads in the preparation of macrobeads have a size range of greater than about 1mm but less than about 6mm in diameter. Preferably, between about 3 and about 4mm in diameter. Ideally about 3.5mm in diameter.

Typically, the beads in the preparation are resistant to the low pH conditions of the stomach.

A further aspect of the invention provides a microbead preparation obtainable by the method as described above.

A further aspect of the invention provides a macrobead preparation obtainable by the method as described above.

A further aspect of the invention provides a microbead preparation comprising a mixture of milk protein product in which the milk protein has at least a 70% degree of denaturation and alginate, wherein the size of the microbeads in the preparation is less than 1000 $\mu$m.

A further aspect of the invention provides a microbead preparation comprising a mixture of milk protein product in which the milk protein has at least a 70% degree of denaturation and alginate, wherein the average size of the microbead in the preparation is from about 300 $\mu$m to about 450 $\mu$m.

A further aspect of the invention provides a macrobead preparation comprising a mixture of milk protein product in which the milk protein has at least a 70% degree of denaturation and alginate, wherein the size of the macrobead in the preparation is >1mm.

A further aspect of the invention provides a macrobead preparation comprising a mixture of milk protein product in which the milk protein has at least a 70% degree of denaturation and alginate, wherein the size of the macrobead in the preparation is from about 3mm to about 4mm.

Typically, the milk protein product is heat-treated or heat-denatured.

Preferably, alginate is sodium alginate. The MPI is as described herein. The alginate is as described herein

A further aspect of the invention provides a food product or beverage product comprising a bead preparation of the invention. The food product may be a dairy product.

A further aspect of the invention provides a pharmaceutical product comprising a bead preparation of the invention. Preferably, the bead preparation is a microbead preparation.

A still further aspect of the invention provides a delivery vehicle for delivery or transport of an active component to a site in the body, comprising a bead preparation of the invention.

Another aspect of the invention provides a method of delivery of an active agent to a site in the body comprising orally administering a preparation of beads of the invention to a subject.

A further aspect provides a method for producing a bead preparation, the method comprising

- providing a solution comprising milk protein product in which the milk protein has at least a 70% degree of denaturation and alginate,
- forming a droplet with said solution, and
- gelation of the droplet in a bath of a solution comprising a cation, to form a bead.

Preferably, the milk protein product is milk protein isolate (MPI). Alternatively, the milk protein product is milk protein concentrate (MPC).

Preferably, alginate is sodium alginate.

Typically, the milk protein product is heat-treated or heat-denatured.

Preferably, the beads are microbeads. Suitably, the beads are macrobeads.

The MPI is as described herein. The alginate is as described herein. The solution is as described herein. The gelation step is as described herein. The step of forming a droplet is as described herein.

*Definitions*

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:

Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein the term "microbead" is understood to mean a substantially spherical droplet of a gelled protein mixture, having an average diameter of about 1 $\mu$m to 1000 $\mu$m.

As used herein the term "macrobead" is understood to mean a substantially spherical droplet of a gelled protein mixture, having an average diameter of greater than about 1mm; preferably between about 1mm to about 6mm; more preferably between about 3mm to 4mm; and ideally about 3.5mm.

The term "active component" when used herein is a compound that has an effect on an organism, tissue or cell.

The term "denaturation" when used herein is a process in which proteins or nucleic acids lose the quaternary structure, tertiary structure and secondary structure or a percentage thereof which is present in their native state. Said protein is referred to as a "denatured" protein or a protein having a particular percentage of denaturation.

**Brief Description of the Figures**

The invention will be more clearly understood from the following description of an embodiment thereof, given by way of example only, with reference to the accompanying drawings, in which: -

Figure 1 is a graph displaying the viscosity of a mixture of 3.1% milk protein (MP) and 2.0% sodium alginate (SA) solutions with different ratios.

Figure 2 illustrates light microscope images of the changes in micro-bead morphology with changes in the ratio of 3.1% milk protein (MP) and 2.0% sodium alginate (SA) solutions. The measurement bar represents 400 $\mu$m size range for all images.

Figure 3 illustrates light microscope images displays the changes of micro-bead morphology with changes in the concentration of milk protein (MP). The measurement bar represents 400 $\mu$m size range for all images.

Figure 4 displays images of macro-beads with different ratios of 3.1% milk protein (MP) and 2.0% sodium alginate (SA) solutions.

Figure 5 displays macro-bead diameter measured by digital slide calliper shown by line and strength of single bead shown by bar as a function of matrix composition (ratio of 3.1% MP and 2% SA solutions). Vertical bar illustrates the standard error in triplicate batches.

Figure 6 is a graph illustrating encapsulation efficiency of folic acid in gel micro-beads with different compositions (3.1% MP/2.0% SA with ratios of 75/25, 70/30, 65/35, 60/40, and 0/100). Vertical bar illustrates the standard error in triplicate batches. Different letters indicate a significant different ($p \leq 0.05$).

Figure 7 illustrates the swelling behaviour of micro-beads based on pure sodium alginate (SA) (■), and MP (3.1%)/SA (2.0%) with ratio of 65/35 (II) evaluated by measuring micro-bead diameter in SGF at pH 3.0 (A), and in SIF at pH 7.0 after 120 min incubation in SGF (B) during incubation in shaking (100 rpm) water bath at 37°C for 180 min.

Figure 8 displays light microscopic images of fresh micro-bead (A), bead incubate in SGF for 120 min (B), in SGF with pepsin for 120 min (C), in SIF for 120 min (D), and in SIF with pancreatin and bile extract for 30 min (E) in shaking (100 rpm) water bath at 37°C.

Figure 9 is a graph illustrating milk protein released from micro-beads consisting of mixture of 3.1% milk protein (MP) and 2.0% sodium alginate (SA) with ratio of 65/35 during 3h incubation in SGF (o), in SGF with pepsin (●), in SIF (□) and in SIF with pancreatin and bile extract (■) in shaking (100rpm) water bath at 37°C. Beads were incubated in SIF and in SIF with pancreatin and bile extract after 120 min incubation of SGF and SGF with pepsin, respectively. Vertical bar illustrates the standard error in triplicate batches.

Figure 10 displays folic acid released from micro-beads consisting of 2.0% sodium alginate (A), and mixture of 3.1% milk protein and 2.0% sodium alginate with ratio of 65/35 (B) during 180 min incubation in SGF (o), in SGF with pepsin (●), in SIF (□) and in SIF with pancreatin and bile extract (■) in shaking (100rpm) water bath at 37°C. Beads were incubated in SIF and in SIF with pancreatin and bile extract after 120 min incubation of SGF and SGF with pepsin, respectively. Vertical bar illustrates the standard error in triplicate batches.

Figure 11 is a schematic diagram of an extrusion device and the process of the extrusion technique.

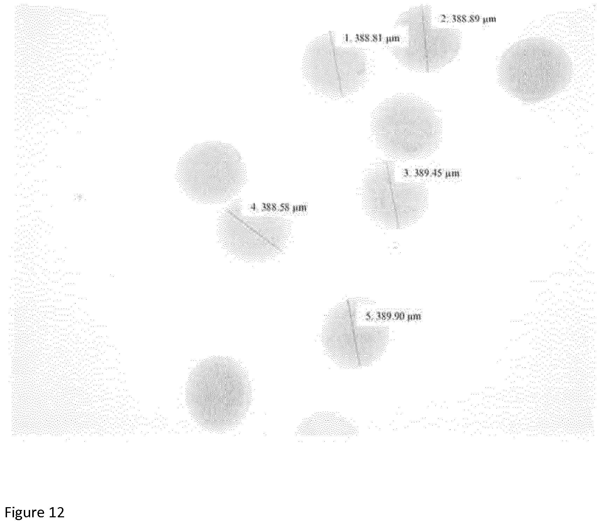

Figure 12 is a light microscopic image of milk protein isolate/sodium alginate microbeads of the invention.

## Details Description of the Invention

In its broadest sense, the invention provides a method for producing beads comprising an active component encased therein, comprising a step of providing a solution comprising denatured milk protein product, alginate and an active component, forming a droplet with said solution, followed by gelation in a bath of a solution comprising a cation. The milk protein product is heat treated. The term bead may be used interchangeably with gel bead. In a preferred embodiment, the beads are microbeads. In another embodiment, the beads are macrobeads. It will be appreciated that the method as described herein may be used for the production of microbeads or macrobeads.

The method of the invention does not involve any steps that expose the beads to high pressure or shear which could damage the active component encased in the bead. This makes the method suitable for producing beads encasing sensitive active components, such as probiotics.

By using milk protein product that has already been subjected to heat treatment, i.e. is denatured, as a component of the beads, the need for a prior heat denaturation step is avoided, unlike prior art methods using whey protein. As well as reducing the number of steps in the method, this improves the viscosity of the mixture which makes it easier to filter if required. Moreover, the inventors have surprisingly found that a smaller amount of heat-treated milk protein product is needed to form the beads compared with using whey protein or whey protein isolate. Heat-treated milk protein product, in particular MPI, also has a high nutritional value, a property which is advantageous for a product with food applications. This component is also widely available and low cost.

Therefore, the method of the invention is easier, cheaper, and less energy intensive process compared to those of the prior art. In this manner, the method of the invention is suitable for large scale production.

In an embodiment, the milk protein product comprises greater than 65% milk protein. The milk protein product comprises at least 60%, 70%, 75%, 80%, 85%, 90%, 95%, 99% or 100% milk protein.

In an embodiment of the invention, the milk protein product is MPI.

MPI is the substance obtained by the partial removal of sufficient non-protein constituents (lactose and minerals) from skim milk to yield a finished product, usually a dry product, containing 80% or more milk protein by weight. MPI is used interchangeably with the term "milk protein (MP)". MPI is generally in the form of a powder.

The MPI comprises 80% or more milk protein. The MPI may comprise 80%, 81%, 82%, 83%, 84%, 85%, 86%, 87%, 88%, 89%, 90%, 91%, 92%, 93%, 94%, 95%, 96%, 97%, 98%, 99% or 100% milk protein. Protein content may be determined by any method known in the art. For example, protein content may be determined by the Kjeldahl method (Kjeldahl method (IDF Standard, 26, 2001) International Dairy Federation).

In an embodiment of the invention, the milk protein product is MPC. MPC is a milk (concentrated) product containing from about 35% to 80% milk protein by weight. MPC may comprise less than 80% milk protein. The MPC may comprise about 35%, 40%, 45%, 50%, 55%,60%, 65%, 70%, or 75% milk protein, preferably at least 65% milk protein. MPC has low molecular weight material, e.g. lactose and small peptides and minerals, and contains casein and whey protein in the same ratio found in milk. Preferably, the MPC does not contain fat.

The milk protein in the milk protein product has a degree of denaturation of at least 60% 70%, 75%, 80%, 85%, 90%, 95% or 99%. Preferably, the milk protein has a degree of denaturation of at least 80%. The milk protein may be completely denatured, i.e. about 100% degree of denaturation. Typically, the whey protein in the milk protein product has a degree of denaturation of at least 60% 70%, 75%, 80%, 85%, 90%, 95%, 99% or 100%.

The milk protein product is heat-treated. Various methods of heat treatment are known in the field. It will be appreciated that any suitable method may be used. Exemplary methods include direct heating, e.g. steam injection, indirect heating, e.g. heat exchanger, or as part of an evaporation and drying process. In one embodiment, the milk protein is heated above 80°C. The heat treatment may be for about 1 second to about 120 seconds, preferably for about 5, 10, 15, 20, 25, 30, 35, 40, 45, 50, 60, 65, 70, 85, 80, 85, 90, 95, 100, 105, 110, 115 seconds. It will be appreciated that the length of the heat treatment may vary depending on a number of factors including but not limited to the temperature and the concentration of the MPI. It will be understood that the length of time is that sufficient to reach the desired degree of denaturation. The heat-treated milk product has a degree of denaturation of at least 70%, 75%, 80%, 85%, 90%, 95% or 99%. Preferably, the heat-treated milk product has a degree of denaturation of at least 80%. The degree of denaturation may be measured by a method known in the art, such as but not limited to, isoelectric precipitation or reversed phase HPLC.

Denaturation may be as a result of pressure treatment, such as high pressure treatment. Methods of denaturation by pressure treatment are known in the art and any suitable method may be used.

The milk protein product is denatured prior to the inclusion in the solution or method of the invention.

In an embodiment, a soluble form of alginate is used. Preferably, the alginate is sodium alginate. Sodium alginate is the sodium salt of alginic acid having the formula $NaC_6H_7O_6$.

The method comprises preparing a solution of milk protein product, alginate and an active component. The milk protein has a degree of denaturation of at least 70%. The milk protein product is heat-treated. In one embodiment, the heat-treated milk product is in the form of a powder. The solution of milk protein product and alginate is referred to as a polymer matrix solution.

A milk protein solution, typically a heat-treated milk protein solution, such as an MPI solution, may be prepared prior to addition of other components. It may be prepared with distilled water. The solution may comprise up to 9% protein to reach protein concentration in the final solution of from about 0.5 % to about 6% w/w. Preferably, the concentration is less than 5% w/w, preferably from about 1.5% to about 3.0 % w/w. Still preferred, the concentration is 2% w/w. Preferably, the concentration is less than 3% w/w. Typically, the concentration is 3% or less w/w.

An alginate solution, e.g. sodium alginate, may be prepared prior to addition of other components. The alginate solution may comprise up to 5% alginate to reach alginate concentrations in the final solution of from about 0.3% to about 3% w/w alginate. Preferably the concentration is from about 0.3% to about 1.5% w/w, preferably from about 0.5% to about 0.8% w/w of the mixture. Still preferred, the concentration is 0.7% w/w of the mixture.

The milk protein product can be dissolved and mixed with alginate to form a polymer matrix solution.

The present invention uses milk protein product, e.g. MPI, as a base and alginate is added to strengthen it, so it still has functionalities governed by protein, not just by alginate. The present invention uses heat-treated milk protein product, e.g. MPI, as a base and alginate is added to strengthen it, so it still has functionalities governed by protein, not just by alginate.

In one embodiment, the polymer matrix solution comprises 2% w/w MPI and 0.7% SA.

The active component is then added to the solution. In an embodiment, wherein the active component is water soluble, *e.g.* folic acid or probiotic bacteria, this component is mixed into the solution of milk protein product and alginate. If the active component is lipid soluble, *e.g.* beta-carotene, vitamins, dyes, flavours, lipids or hydrophobic peptides, such components may be emulsified in oil and an emulsifier solution before being mixed into the milk protein product/alginate solution. MPI may be used as an emulsifier. It will be appreciated that emulsification can be achieved using any standard homogeniser.

It is to be understood that the components of the solution or polymer matrix solution may be added in any order.

The solution is then treated to form a droplet. Various methods of generating a droplet will be known to a person skilled in the art. The droplet may be produced by extrusion. In one embodiment, the method comprises extrusion of the solution from a syringe through a vibrating nozzle. In such a method, the solution is extruded though a nozzle and breakup of the jet is induced by applying a sinusoidal frequency to the nozzle.

Alternatively, the droplet is produced by jet-cutting. In this method droplet generation is achieved by cutting the jet of fluid coming out of a nozzle by means of a rotating cutting wires into cylindrical segments which then form beads or droplets due to the surface tension on the passage to the bath.

An exemplary device for undertaking extrusion is the Büchi Encapsulator B-390, the Nisco encapulsation system or the BRACE encapsulation system. An equivalent device may be used.

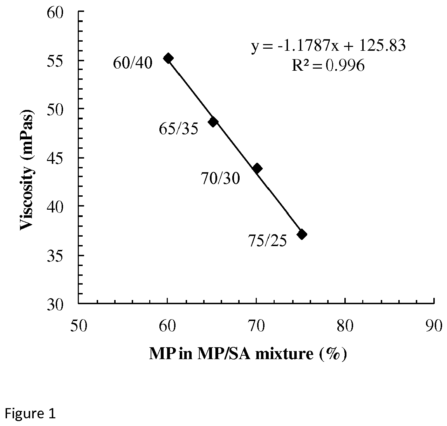

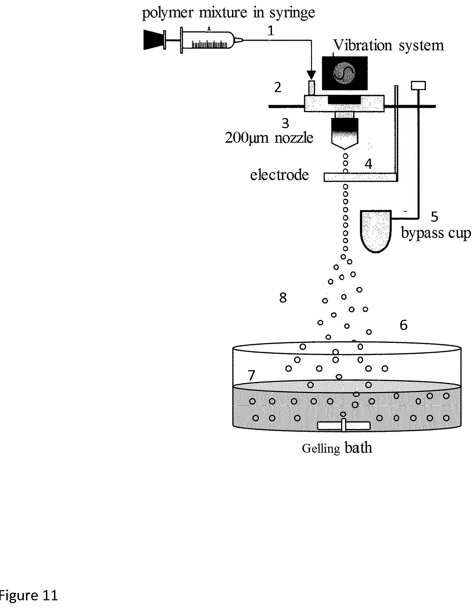

The process of the extrusion technique using an encapsulator device is illustrated by Figure 11. As illustrated by this Figure, the suspension is delivered to the nozzle via a feed-line 1 which is connected to the polymer reservoir. As show in Figure 1, the diameter of the aperture of the nozzle is $200 \mu m$. The nozzle (3) is connected via a PTFE membrane, to a vibrating device (2), which is insulated from the surrounding structures by rubber mounts to avoid the generation of resonance frequencies in the system. The flow of solution to the nozzle (3) is accomplished using a precision syringe pump with maximum extrusion volume of 50 ml. The solution 8 is extruded through the nozzle and passed through an electrode (4) into a gelling bath (6) containing a crosslinking solution (7). A collection cup, suspended (5) from the top plate, was utilized during the initial priming of the nozzle with protein-probiotic mixture. This facilitates the retrieval of initial polymer droplets with a diameter in excess of the predicted value (defined under controlled conditions) and thus ensured monodispersity of the subsequent microbead batch. It will be understood that this is exemplary only.

It will be appreciated that this device may also be used for macrobead production.

It will be appreciated that any combination of parameters of the device may be used and such combinations depend on nozzle size and viscosity of the polymer solution. For instance, if the polymer solution is very viscous which may be caused by a high sodium alginate and protein content, the nozzle size required will be larger and flow rates will increase. A person skilled in the art may visually optimize parameters to create a laminar flow and small droplets. It will be appreciated that the frequency, the flow rate and the amplitude can be controlled as desired by the user and calculated by any suitable means known in the art.

In an embodiment of the invention, the spray nozzle has an aperture of from about 100 $\mu m$ to about 1mm in diameter. Preferably, said aperture is from about 150 $\mu m$, to 400 $\mu m$. Still preferred, said aperture is ideally 200 $\mu m$. It will be understood that the following values are for a nozzle having a 200 $\mu m$ diameter. However, it will be appreciated that the values may equally apply to a nozzle of a different size.

The frequency of the vibrating nozzle is from about 1000 Hz to 4000 Hz. In a preferred embodiment, the frequency is from about 1200Hz to 2000Hz, ideally, from about 1500, 1600, 1700 or 1800 Hz.

The flow rate is from about 3.5 to about 6.5 ml/min. In a preferred embodiment, the flow rate may be 4.6, 4.7. 4.8, 4.9, 5, 5.1, 5.2, 5.3, 5.4, 5.5, 5.6, 5.7, 5.8, 5.9 or 6ml/min. Ideally, the flow rate is 5.7 ml/min.

The amplitude range is from about 1 to 7. Preferably the range is 3, 4, 5, ideally 6.

The distance between the nozzle and the gelling bath is called the falling distance. In a typical embodiment, the falling distance is 25cm or less, preferably about 8cm, 9cm, 10cm, 11cm, 12cm, 13cm, 14cm, 15cm, 16cm, 17 cm, or 18cm.

The solution is extruded through the nozzle and passed into a bath or gel bath containing a crosslinking solution comprising a cation. Any suitable cation may be used. In one embodiment, the cation is selected from the group comprising magnesium, iron and calcium chloride. In a preferred embodiment, the cation is calcium chloride.

In a preferred embodiment, the solution comprises from about 0.1M to about 0.5M calcium chloride. Preferably, from about 0.15, 0.2, 0.25 or 0.3M calcium chloride, preferably about 0.2M calcium chloride.

The solution may be stirred or agitated during gelation. This allows the formation of uniform spherical microbeads.

In one embodiment, the solution may comprise an effective amount of a surfactant. The added surfactant is preferably an effective amount of Tween-20. In a typical embodiment, from about 0.01% to 0.1 % w/w, Tween-20 is added, preferably, about 0.05% w/w. This prevents agglomeration of the microbeads and imparts stability of single microbeads in solution.

Gelation is carried out for about 15 to about 60 minutes, preferably with agitation. In a preferred embodiment, this step is about 20 mins, 25 mins, 30 mins, 35 mins, 40 mins, 45 mins, 50 mins or 55 mins. It will be appreciated that the time taken for gelation is a time sufficient to effectively cure or crosslink the microbeads in the preparation.

In one embodiment of producing a preparation of macrobeads, the droplet may be formed with a nozzle having an aperture of from about 0.5mm to about 2.5mm, preferably from 1mm or larger. The droplet may be produced using a pipette, a burette, or a syringe, having an aperture of from about 0.5mm to about 2.5mm, preferably from 1mm or larger.

In an embodiment of the invention, the solution may be filtered prior to formation of the microbeads. In one embodiment, the solution is filtered through a large pore filter. Typically, the filter has pores between about 5$\mu m$ and 150 $\mu m$. The pore size is typically smaller than the nozzle sized used in the method. This removes any undissolved material. In one embodiment, the solution is filtered through a 10 $\mu m$ membrane syringe filter e.g. 10.0 $\mu m$ Versapor $^{®}$ membrane syringe filter, prior to extrusion through the nozzle.

After gelation, the microbead preparation is then recovered. Optionally, the microbead preparation may be washed to remove calcium. The preparation may be washed with water.

A further aspect of the invention provides a microbead preparation produced by the method of the invention.

A further aspect of the invention provides a microbead preparation comprising a mixture of milk protein product, typically heat-treated milk protein product and alginate. The milk protein in the milk protein product has at least 70% degree of denaturation. Preferably, the size of the microbead in the preparation is from about 300 $\mu$m to about 450 $\mu$m. Preferably, the average size of the microbead in the preparation is from about 300 $\mu$m to about 450 $\mu$m. The microbead may have an active component encased therein.

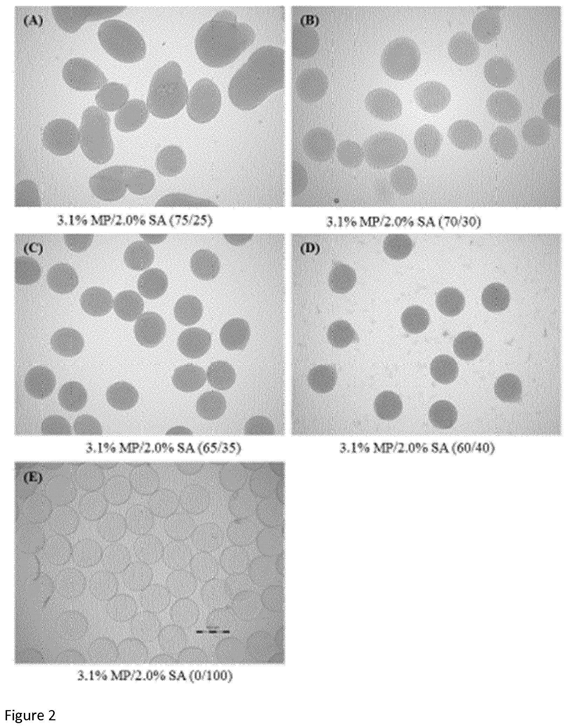

The microbeads in the preparation(s) of the invention have a diameter of from about 150$\mu$m to about 500$\mu$m; and preferably from about 300 $\mu$m to about 450 $\mu$m. preferably 310, 320, 330, 340, 350, 360, 370, 380, 390, 400, 410, 420, 430, 440, 450 $\mu$m. Typically, each microbead in the preparation has a diameter of from about 300 $\mu$m to 450 $\mu$m. In one embodiment, at least 70%, 80% or 90% of the microbeads have a diameter of from about 300 $\mu$m to 450 $\mu$m. Figure 2 is a light microscopic image of MPI/sodium alginate microbeads of the invention. Size (diameter in $\mu$m) can be estimated by microscopy and calibrated image analysis, or similar method known in the art.

The macrobeads in the preparation of macrobeads have a size range or diameter of greater than about 1mm but less than about 6mm in diameter. Preferably, from about 1.5mm to about 5.5mm, typically, about 2mm, 2.5mm, 3mm, 3.5mm, 4mm, 4.5mm, or 5mm in diameter. Ideally about 3.5mm in diameter. Typically, each macrobead in the preparation has a diameter of from about 3 to 4mm. In one embodiment, at least 70%, 80% or 90% of the microbeads have a diameter of from about 3 to 4mm. The size may be the average size.

The beads of the preparation of the invention are robust and capable of surviving intact in the low pH of the stomach and releasing the active agent at a higher pH. When tested the beads of the invention are stable at low pH compared to prior art microbeads such as alginate based microbeads, without any disintegration at the conditions mimicking the upper gastro intestinal tract. Therefore, the preparation is capable of acting as a vehicle to protect an active agent in the stomach and allow its passage through the stomach after ingestion. The beads in the preparation can disintegrate at the more alkaline environment of the small intestine. Therefore, the bead of the invention is capable of protecting the encapsulated component such as folic acid in the acidic gastric condition and release it in the alkaline condition in the small intestine. This is beneficial as absorbance of vitamins takes place in the small intestine. This allows the release and delivery of the active agent to this area. The bead of the invention allows sustained release of the component encased therein.

Using milk protein as a component of the microbeads of the invention has an advantage in terms of protection and control release of active components during passage through gastro-intestinal tract but may also provide essential bioactive peptides from hydrolysis of milk protein by digestive enzymes which may exert a number of physiological effects.

The beads are capable of surviving intact in simulated gastric fluid (SGF) of pH 3. The beads rupture in simulated intestinal fluid (SIF) at pH 7 within 30 mins, preferably in the presence of digestive enzymes.

To this end, a further aspect of the invention provides a method of delivering an active agent comprising orally administrating an effective amount of the microbead preparation. The microbead preparation may be administered directly, in a capsule and/or as an ingredient in a food product or beverage.

The active agent or component is as described herein. In one embodiment, the bioactive may be a bioactive peptide produced by hydrolysis of the milk protein by digestive enzymes.

A further aspect of the invention provides a food product or a beverage product comprising the microbead preparation of the invention. It will be understood that the food product may be any such product known in the art, for example, a dairy product, such as milk, cheese or yogurt, bars e.g. energy or protein bars. Similarly, the beverage product may be any beverage product known in the art.

## EXAMPLES

## Materials and Methods

### Materials

Heat treated milk protein isolate (MPI, 87% protein, w/w) was provided by Kerry ingredients (Kerry Group, Ireland). Its protein content (87%) was determined by the Kjeldahl method ((IDF Standard, 26, 2001) using a nitrogen-to-protein conversion factor of 6.38). Sodium alginate (SA) was obtained from Büchi (Büchi Labortechnik AG, Switzerland). Folic acid ($\geq$ 97%), sodium hydroxide, calcium chloride dehydrate, potassium chloride, potassium dihydrogen phosphate, sodium bicarbonate, sodium chloride, magnesium chloride hexahydrate, sodium citrate, pepsin (extracted from porcine stomach mucosa, activity: 837 U/mg of protein), pancreatin (extracted from porcine pancreas, 4xUSP specification), bile acid (porcine bile extract) were purchased from Sigma-Aldrich (Dublin, Ireland). Tween-20 was obtained from BDH (VWR International Ltd., Dublin, Ireland). The chemical products used in high performance liquid chromatography (HPLC) were acetonitrile (ACN) and trifluoroacetic acid (TFA), both HPLC grade purchased from Fisher Scientific Ltd., (Dublin, Ireland). Milli-Q water (Millipore, Cork, Ireland) was used for HPLC analysis.

### Encapsulating matrix solution preparation

The solutions of MPI (5.0-8.0%, w/w) were prepared using distilled water. The solution was stirred for at least 3 hrs at room temperature. MPI solutions were adjusted at pH 7.0 with 0.5 M sodium hydroxide and then allowed to stand for overnight at 4°C in cold room to ensure complete hydration of proteins. Following overnight storage, the solutions were brought to room temperature with further stirring for at least 45 min and final checked at pH 7.0. Solutions were subjected to centrifugation in sealed bottles at 14,000 rpm for 30 min at 20°C using SLA 1500 rotar (SORVALL ® RC PLUS, Thermo electron LED GmbH, D - 63505 Langenselbold, Germany). The supernatant was immediately transferred after centrifugation into separate beaker for each solution. The final protein content in each solution was estimated (2.7, 3.1, 3.5 and 4.1% protein in supernatant of 5.0, 6.0, 7.0, and 8.0% MPI solutions, respectively) by Kjeldahl method and Quick Start Bradford Protein Assay kit. The solution of SA (2.0%, w/w) was prepared with distilled water and stirred gently overnight at room temperature. Sodium azide (Sigma Chemical Co., S. Louis, MO, USA) can be added at a final concentration of 0.05% (w/w) to SA solution as an antimicrobial agent. Following overnight stirring, alginate solution was filtered through 5.0 $\mu$m Minisart syringe filter (Sartorius Stedim Biotech GmbH, Göttingen, Germany) and stored at room temperature for further use up to 2 weeks.

MP (3.1%) and SA (2.0%) solutions were combined with ratios of 75/25, 70/30, 65/35, and 60/40 to make polymer matrix solutions. Polymer matrix solutions were also prepared using solutions of MP (2.7, 3.5 and 4.1%) and SA (2.0%) with ratio of 65/35. Viscosity of polymer solutions was measured using an AR 2000ex Rheometer (TA Instruments UK, Ltd., Crawley, England) with a cone and plate configuration at a constant shear rate of 400 s$^{-1}$ at control temperature (20°C) to find out viscosity suitable for Encapsulator. Polymer matrix solutions were filtered through 10.0 $\mu$m Versapor ® membrane syringe filter (PALL Life Sciences, Ann Arbor, MI, USA) prior to extrusion through the nozzle.

### Microbead preparation

Microbeads were prepared by extrusion method using the Inotech IE-50R Encapsulator ® (Inotech AG, Dottikon, Switzerland) as described by Doherty et al, (Food Hydrocolloids, 2011;25:1604-1617.). Each polymer solution was extruded through a vibrating nozzle (diameter 200 $\mu$m) under frequency of 1500-1800 Hz with flow rate of 5.7-5.8 ml/min into a calcium chloride (0.2 M) gelling bath, containing 0.05% Tween-20 with continuous slow stirring. Micro-beads prepared from SA solution was used as reference. Micro-beads were cured/polymerised in gelling bath for 30 min. The amount of protein leakage out from mixed MP/SA during polymerisation was evaluated by determining the amount of protein leakage into the calcium chloride gelling bath using Quick Start Bradford Protein Assay kit.

The polymer matrix solutions prepared with combination of MP (3.1%) and SA (2.0%) solutions with ratios of 75/25 (2.325%MP/0.5% SA), 70/30 (2.17%MP/0.6% SA), 65/35 (2.015% MP/0.7% SA) and 60/40 (1.86% MP/0.8% SA) were used for encapsulation of folic acid and their encapsulation efficiency into the beads. Folic acid was added at a final concentration of 1 mM in each polymer solution.

### Macro-bead preparation

Macro-beads were prepared using polymer matrix solutions of MP (3.1%)/SA (2.0%) with ratios of 75/25, 70/30, 65/35, and 60/40 by manually dropping using a Pasteur pipette into 0.2 M CaCl$_2$ gelling bath containing 0.05% Tween-20 with continuous slow stirring. Beads were cured/polymerised in gelling bath for 30 min.

### Bead size and morphology

The size and morphology of micro-beads were analysed using an optical microscope equipped with a digital camera (BX51 light microscope, Olympus, Essex, UK). For each formulation, diameter of randomly selected 25 micro-beads was measured using a scale bar with x10 magnification. The diameter of randomly selected 25 macro-beads was measured by digital calliper gauge (Work Zone, GT-DC-02, ALDI Stores Ireland Ltd). The size of each formulation was presented as the mean size $\pm$ standard deviation (SD) of triplicate batches.

### Mechanical strength

The mechanical strength of MP/SA macrobeads (for each formulation) was analysed using a texture analyser (TA-HDi, Stable Micro Systems, Godalming, UK). A specific force was applied to a macro-bead and the quantity of deformation/rupture of the bead was assigned as a measure of mechanical strength. Strength assay was performed using a 20mm diameter cylindrical aluminium probe at a mobile speed of 0.3 mm/s in compression mode. A rupture distance of 95% was applied and the peak force (expressed in gram force) exerted by the probe on the bead was recorded. Analysis was conducted on separate 10 single bead per batch and 3 replicate batches were analysed. The results were expressed as the mean $\pm$ standard deviation (SD) for triplicate batches.

Encapsulation efficiency

Microbeads of SA and MP (3.1%)/SA (2.0%) with ratios of 75/25, 70/30, 65/35, and 60/40 with folic acid were washed with distilled water and then dried by blotting with tissue paper. Dried microbeads (0.5 mg) were accurately weighed and dispersed in 10 ml of 50 mM sodium citrate solution at pH 7.5 in 15 ml falcon tube (120x17mm) (Sarstedt, Germany). Tubes were wrapped with aluminium foil to protect folic acid from light during extraction procedure. The solutions were then diluted in appropriate concentration with distilled water and filtered through 0.2 $\mu$m syringe filters prior to analysis of folic acid by HPLC method. The amount of folic acid leakage out from SA, and MP/SA polymer matrix solutions into the calcium chloride gelling bath during the curing of micro-beads was also determined. Folic acid content was determined using reverse-phase HPLC with C18 column. Encapsulation efficiency (EE) of folic acid was calculated as follows:

$$EE\ (\%) = \frac{\text{Amount of folic acid in micro} - \text{beads}}{\text{Amount of folic acid loaded in polymer solution}} \times 100$$

Simulated gastro-intestinal (GI) study

SA and MP (3.1%)/SA (2.0%) micro-beads with and without folic acid were incubated in simulated gastric fluid (SGF) at pH 3.0 and in simulated intestinal fluid (SIF) at pH 7.0 in the presence and absence of digestive enzymes. SGF media was formulated according to slight modification as reported by Minekus et al., (2014) and consisted of 6.9 mM potassium chloride, 0.9 mM potassium dihydrogen phosphate, 25 mM sodium bicarbonate, 47.2 mM sodium chloride, and 0.1 mM magnesium chloride hexahydrate. The pH of the SGF 3.0 was adjusted using 1.0 M hydrochloric acid. Simulated gastric digestion studies were performed with and without pepsin. Pepsin was added into SGF to achieve 1000 U/ml in the final digestion mixture. Samples (1.0 g beads with 9.0 ml water and 10.0 ml SGF with or without pepsin) were incubated in water bath at 37°C under agitation (100 rpm) for up to 180 min. The pH 3.0 of the digestion mixture was adjusted prior to incubation. Samples were recovered after pre-determined time (30, 60, 90, 120, and 180 min) interval during 180 min incubation. Pepsin activity was inactivated by neutralising the samples using 0.5 M sodium hydroxide and subsequently analysed protein content in micro-beads and in digestion fluid, and folic acid released in the fluid during incubation. For intestinal study, micro-bead samples digested in gastric media for 120 min were mixed with SIF (ratio of gastric phase to SIF of 50/50, v/v) and subsequently incubated at 37°C with agitation (100 rpm). Simulated intestinal fluid consists of 6.8 mM potassium chloride, 0.8 mM potassium dihydrogen phosphate, 85.0 mM sodium bicarbonate, 38.4 mM sodium chloride, and 0.33 mM magnesium chloride hexahydrate. The pH 7.0 of intestinal digestion mixture was adjusted using 1.0 M sodium hydroxide/hydrochloric acid prior to incubation. Simulated intestinal digestion was performed in the presence and absence of bile (10 mM in final mixture) and pancreatic enzymes. The amount of pancreatin was added to achieve trypsin activity of 100 U/ml of final mixture. Samples were recovered after pre-determined time (30, 60, 90, 120, and 180min) interval during 180 min incubation. Enzymes were inactivated immediately after recovering the sample using heat treatment in water bath at 95°C for 30sec. The pH was also re-adjusted using 0.5 M HCl during digestion. Individual sample bottles were used for each time point. Three independent studies were conducted and results were expressed using the mean value $\pm$ SD.

Swelling behaviour

Swelling behaviour of SA and MP/SA (65/35) microbeads were evaluated in simulated gastric fluid (pH 3.0) and intestinal fluid (pH 7.0) in the presence and absence of digestive enzymes. Micro-beads from GI assay were recovered at pre-determined time (30, 60, 90, 120, and 180 min) intervals during gastric and intestinal incubation. Diameter of randomly selected 25 micro-beads were analysed by light microscope at a magnification of x10. Swelling behaviour (%) was calculated as follows:

$$Swelling\ (\%) = \frac{\text{Diameter of microbeads after incubation} - \text{Initial diameter}}{\text{Initial diameter of microbeads}} \times 100$$

Protein assay

Degradation of MP/SA (65/35) microbeads was investigated by following protein released into SGF (pH 3.0) and SIF (pH 7.0) in the presence and absence of digestive enzyme during incubation at 37°C. Protein content in microbeads during digestion in the presence of digestive enzymes was also analysed. In this case, microbeads recovered at each time point were washed with distilled water and then dissolved into 50 mM sodium citrate at pH 7.5. Samples were filtered through 0.45 $\mu$m syringe filter prior to analysis. Protein content was analysed spectrophotometrically using Quick Start Bradford Protein Assay kit.

Size exclusion chromatography

Size exclusion chromatography (SEC) was performed on MP/SA micro-bead samples incubated in simulated GI studies using 2695 Waters ™ HPLC system (Millipore, Middlesex, UK) equipped with a UV/Visible detector (waters 2489) and a TSK G2000 SW column (600 x 7.5 mm; Tosu Hass, Japan) according to the method outlined by Doherty et al., (2010). The samples were eluted using 30% ACN containing 0.1% TFA (v/v) at a flow rate of 1 ml/min. A molecular weight calibration curve was prepared from the retention time of standard proteins and peptides. All samples were filtered through 0.2 $\mu$m syringe filter prior to injecting into the HPLC system.

Folic acid assay

Folic acid in standard solutions, fresh micro-beads, and folic acid released in GI fluid in the presence and absence of digestive enzymes during incubation of micro-beads at 37°C was determined using reverse-phase 2695 Waters ™ HPLC system (Millipore, Middlesex, UK) equipped with a UV/Visible detector (waters 2489) and a Phenomenex Jupiter C18 (4.6 mm x 250 mm x 5 $\mu$m, 300 Å, Phenomenex, Cheshire, UK) reverse phase column. A gradient of solvent B and solvent C (at 86.7: 13.3 for 5 min, from 86.7:13.3 to 72.2: 27.8 in 15 min, from 72.2: 27.8 to 0.0: 100.0 in 2 min, at 0.0:100.0 for 5 min, from 0.0: 100.0 to 86.7: 13.3 in 2 min and at 86.7: 13.3 for 5 min) was used as mobile phase at a flow-rate of 1 ml/min, where Solvent B was 0.1% TFA (v/v) in Milli-Q water and solvent C was 90% acetonitrile (MeCN) containing 0.1% TFA (v/v). Wavelength of detection was 214 and 290 nm. An injection volume of 20 $\mu$l was loaded onto the column and column temperature was 28°C.
Folic acid content was calculated using standard curve prepared with 5 different concentrations (from 1.0 to 20.0 $\mu$g/ml) of standard folic acid. Folic acid ($\geq$ 97%) of 10 mg was accurately weighed in 100 ml volumetric flask. Initially 50 ml of phosphate buffer (50 mM, pH 7.0) was added into the flask and folic acid was completely dissolved by gentle shaking. Phosphate buffer was added to volumetric flask up to the volume mark and mix properly. This solution was used as stock standard. Five different concentrations of working standard were prepared from this stock solution to make standard curve. The solutions were filtered through 0.2 $\mu$m syringe filter prior to injecting into the HPLC system.

Statistical analysis

All experiments were performed at least in triplicate. Mean results are presented, and vertical error bars on graph represent standard deviation. A 1-way ANOVA followed by multiple range (LSD) tests were performed using SPSS 15.0 (SPSS Inc., Chicago, Ill., U.S.A.) to compare data values. The level of confidence required for significance was selected as $p \leq 0.05$.

Assessment of microbead formulation for its suitability as a delivery system for probiotic cultures.

A bottle containing the two formulations [3.1% MP solution and 2% Na-alginate solution at a ratio of 65/35; prepared as described above] was poured into a second bottle containing an amount of centrifuged probiotic bacterial culture [such as Lactobacillus rhamnosus (LGG®)] to reach a final concentration of $1 \times 10^9$ CFU/mL in the mixture. The probiotic/polymer mixture was then agitated gently using a magnetic stir bar and stir plate. Typical amounts would be 100-500mL.
The formulation and the probiotic mixed well to form a homogeneously dispersion. The formulation mixture was passed through a filter prior to extrusion through a 300 $\mu$m nozzle and formed good spherical beads once it entered the gelling solution of 0.2 M CaCl$_2$ containing 0.05% Tween-20. The formulation was easy to work with and remained fluid throughout the encapsulation process. This was repeated on two further occasions within the same hour using the same dilution factor to generate 300 mL of beads.
An additional formulation mixture was prepared whereby a higher amount of the probiotic culture ($3.3 \times 10^{10}$ CFU/mL in the final mixture)] was mixed with the formulation. This enabled an understanding of how the formulation could work with higher loading of the beads. As was seen in the previous example, dilution factor the formulation was easy to work with and passed through the filter followed by the 300 $\mu$m nozzle. The beads that were formed in the hardening solution and to the naked eye looked to be stronger than the previous batch. On the day of preparation the total bacterial load in the premix and one batch of beads was $3.3 \times 10^{10}$ CFU/mL. The beads were again assessed after 1 week storage at 4°C with 2.01 $\times 10^{10}$ CFU/mL, i.e. a small loss in viability.

**RESULTS**

Matrix solution and microbead preparation

MPI solution at concentrations of > 8.0% was very viscous without instant gelation behaviour. When milk proteins are thermal processing for heat denaturation, κ-casein of casein fraction interact with the heat denatured unfolded whey proteins by hydrophobic interaction and/or disulphide bond. This bond has a protective effect against heat-induced aggregation of whey proteins, which in turn reduced the aggregate size and gelling behaviour of milk protein. The solutions of MPI (≤ 8.0%, w/w) were combined with solution of SA (2.0%, w/w) to increase their gelation behaviour. MPI solutions were centrifuged prior to mix with SA solution to remove any unhydrated proteins, which make matrix solutions very viscous immediate after mixing with SA solution. The final protein content in each solution was estimated 2.7, 3.1, 3.5 and 4.1% in supernatant of 5.0, 6.0, 7.0, and 8.0% of MPI solutions, respectively. The polymer matrix solutions from mixture of MP (from 3.5 to 4.1%) and SA solutions with ratios of 65/35 and 60/40 generated rigid gel structure immediate after mixing. Matrix solutions from mixture of MP (from 3.5 to 4.1%) and SA solutions (2.0%) with ratios of 75/25 and 70/30 were easy to extrude through 200 μm vibrating nozzle. Mixtures of MP (from 2.7 to 3.1%) and SA solutions (2.0%) with ratio of 65/35 and 60/40 generated suitable polymer matrix solution with instant gelation behaviour. A steady stream of matrix droplets was extruded through the nozzle from these solutions representing the preliminary requirement to prevent flocculation of polymer droplets upon contact with calcium chloride curing media. Viscosity of polymer matrices was also an important factor to extrude through the vibrating nozzle for micro-bead preparation. Figure 1 shows that viscosity of the polymer solutions increased linearly with increase of SA in the mix. But instant gelation behaviour was also increased with increasing SA. Matrix solutions with viscosity values between 48 and 55 mPas were found most suitable polymer matrices with instant gelation behaviour to extrude through 200 μm vibrating nozzle for preparation of micro-beads.

Micro-bead size and morphology

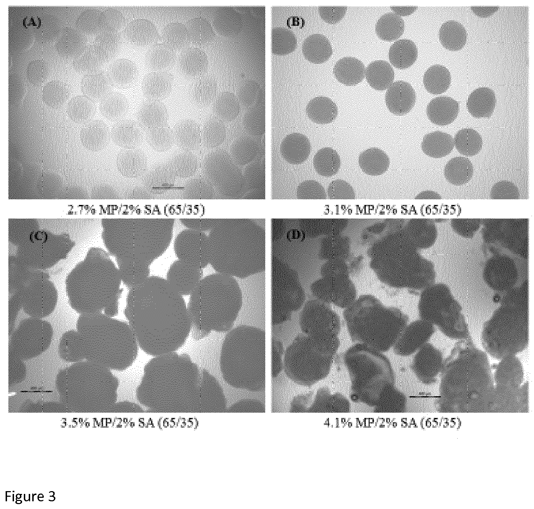

Light microscopic images of micro-beads manufactured by extrusion method using various polymer matrices are shown in Figure 2 and Figure 3. Microscopic images showed that shape and size of micro-beads were varied according to composition of polymer matrices. Flocculated non-spherical shape beads with a wide range of size distribution were obtained from MP (3.1%)/SA (2.0%) ratios of 75/25 and 70/30 (Figure 2A and 2B). Irregular shape with aggregated large beads was also observed from matrix solutions of MP (3.5-4.1%)/SA (2.0%) with ratio of 65/35 (Figures 3C and 3D). Mostly homogeneous and spherical beads with average diameter of 388.4 ± 14.7 μm and 390.2 ± 10.5 μm were obtained from formulation of MP (3.1%)/SA with ratios of 65/35 (Figure 2C) and 60/40 (Figure 2D), respectively. Spherical with some flocculated beads with average diameter of 379.6 ± 18.7 μm were obtained from formulation of MP (2.7%)/SA with ratios of 65/35 (Figure 3A). Micro-beads manufactured from only SA solution (2.0%) for use as a reference was homogeneous with narrow size range (360.9 ± 5.4 μm in diameter) (Figure 2E). Slow gelation behaviour of polymer solutions with relatively lower percentage of SA in 75/25 and 70/30 formulation could be caused for formation of flocculated non-spherical beads. On the other hand, rigid gel stricture of formulation containing high percentage of SA with high protein content (3.5-4.1%) (ratios of 65/35 and 64/40) could be responsible for formation of irregular shaped beads. Although instant gelation of polymer droplets occurred in calcium chloride curing media however there was certain protein leakage (11-12%) was detected in curing media during micro-beads preparation using polymer matrix with ratio of 65/35.

Size and strength of macro-bead

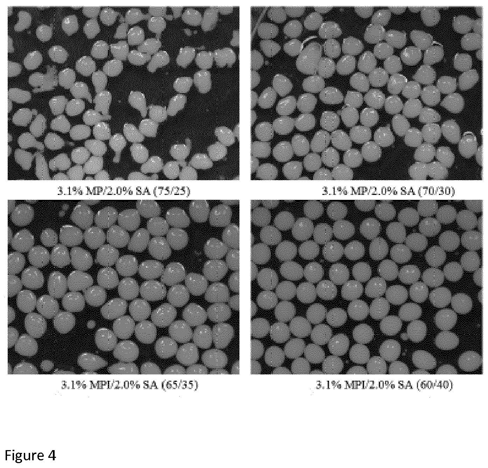

The mechanical strength of gel beads is very important for handling, processing and stability study. To measure the mechanical strength of gel beads it was important to have individual or same number of beads on every measurement time. Since handle of individual or same number of microbeads for every experiment was difficult therefore macrobeads were prepared to measure the mechanical strength of gel beads by manually dropping of polymer solutions using a pasteur pipette with the same formulations (3.1% MP and 2.0% SA solutions with ratios of 75/25, 70/30, 65/35, 60/40 and 0/100) used for preparation of micro-beads. Images of macrobeads (Figure 4) showed that sphericity of macro-beads was similar with micro-beads. Sphericity of beads increased with increase of SA concentration in the matrix solutions. Beads sphericity mainly depends on gelation behaviour of matrix solution. Instant gelation behaviour of matrix solutions with ratios of 65/35 and 60/40 produced mostly spherical micro-beads.

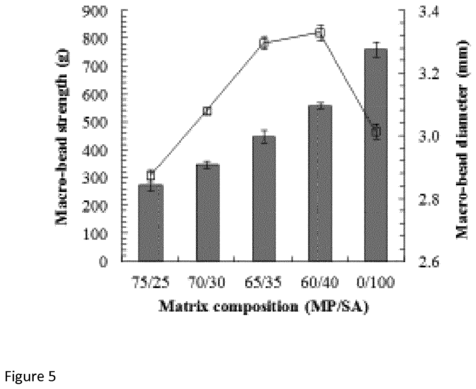

Figure 5 showed that size and mechanical strength of gel macro-beads increased with increasing SA content in the formulation. For example, lowest size and mechanical strength of beads was obtained when alginate content was lowest (25%) in the formulation, whereas highest size and mechanical strength of beads was found when alginate content was highest (40%) in the formulation of mixture of MP and SA solutions. However, highest mechanical strength of pure SA macro-bead (MP/SA, 0/100) was found though bead size was lower than MP/SA with ratio of 70/30. There was no significant difference in bead sizes prepared from formulation of 65/35 and 60/40, but mechanical strength was lower for macro-bead prepared from formulation of 65/35 than for beads prepared from formulation of 60/40. Data showed that SA concentration in the matrix solutions not only affected beads sphericity but also beads size and strength.

Encapsulation of folic acid in gel micro-beads

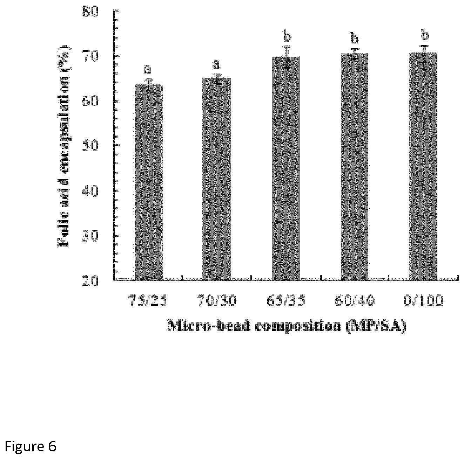

Encapsulation efficiency (EE) of folic acid in gel micro-beads manufactured using matrix solutions of MP (3.1%)/SA (2.0%) with ratios of 75/25, 70/30, 65/35, and 60/40 are shown in Figure 6. Folic acid was also encapsulated in pure SA (2.0%) micro-beads to use as reference (MP/SA, 0/100 in Figure 6). Data showed that EE of folic acid in gel micro-beads increased with increase of SA concentration in the matrix solutions. Encapsulation of folic acid in SA micro-beads and in MP/SA micro-beads with ratios of 65/35 and 60/40 was significantly higher than in MP/SA micro-beads with ratios of 75/25 and 70/30. There was no significant different in EE between SA beads and MP/SA beads with ratios of 65/35 and 60/40. Encapsulation of folic acid mainly occurred by physical entrapment in polymer matrices, there was no any chemical interaction between folic acid and polymer matrix components. Due to slow gelation of polymer solutions with ratios of 75/25 and 70/30, water soluble folic acid may diffuse from the matrix solutions to the calcium chloride curing media before formation of complete gel beads and loss in entrapment efficiency. On the other hand polymer solutions with ratios of 65/35 and 60/40, where instant gelation of polymer droplets occurred and formed spherical beads in curing media, there was comparatively little chance for folic acid diffusion from matrix solutions to the calcium chloride curing media. However, overall folic acid encapsulation in gel micro-beads was relatively high in all formulations (63-71%). Similar encapsulation efficiency of water soluble bioactive, such as riboflavin was also achieved in alginate-whey protein microspheres by Chen and Subirade (2007) and in whey protein microgel by Egan et al., (2014). Although around 100% encapsulation yield of probiotic bacteria was achieved in whey protein micro-beads by Doherty et al., (2011) and in alginatemilk microspheres by Shi et al., (2013). Doherty et al., (2011) observed micro-beads by image analysis and reported that micro-beads produced by extrusion method possessed large pore sizes with uneven surface. Higher encapsulation yield of probiotic bacteria was possibly due to larger size of bacteria cannot diffuse through the pores of beads surface. In contrast, low molecular weight water soluble bioactives such as folic acid or riboflavin may be allowed for diffusion through the pores of bead surface during the curing/polymerisation of bead, as a result comparatively low encapsulation yield for folic acid than probiotic bacteria. Moreover, ringing of microbeads prior to estimation of folic acid could also be caused for losses of water soluble folic acid from all formulations.

Simulated gastro-intestinal incubation

The physico-chemical changes of microbeads produced from formulation of MP (3.1%)/SA (2.0%) with ratio of 65/35 were investigated during incubation in SGF and SIF with and without digestive enzymes. Pure SA beads were also used as reference. The changes of microbeads diameter during incubation in GI fluid were used as indication of shrinking or swelling of beads, and MP released from MP/SA was used as indication of micro-beads degradation. Since MP and SA are pH dependent polymers, therefore we focused on these two parameters during GI incubation for beads stability purposes.

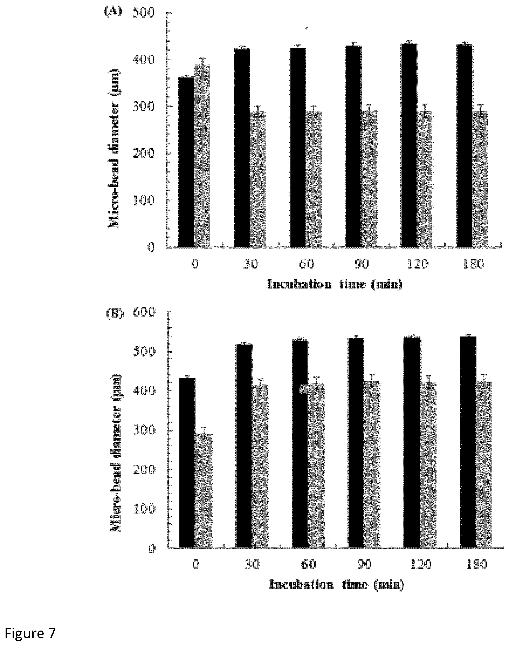

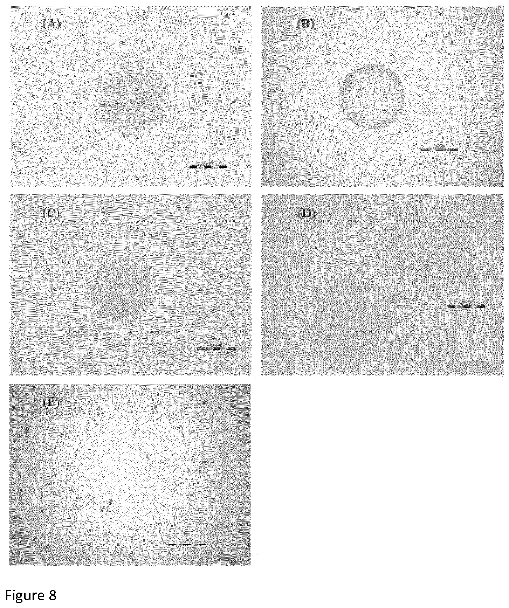

Pure SA microbeads were swelled with 19% increase in diameter when incubated in SGF at pH 3.0 in absence of pepsin at 37°C (Figure 7A). In contrast, a significant shrinkage of MP/SA micro-bead was observed when incubated in SGF at pH 3.0 in absence of pepsin, which translated to approximate 25% decreased in diameter on following 180 min incubation at 37°C (Figure 7A), whereas 24% decreased in diameter was observed in presence of pepsin (data not shown). SA beads were not incubated in SGF with pepsin, because of no protein present in alginate beads. Swelling of SA beads, and shrinking of MP/SA were occurred mostly during the initial 30 min of gastric incubation. Light microscopic images of MP/SA fresh micro-beads, and shrinkage micro-beads during gastric incubation are shown in Figure 8, which revealed contractile surface of the beads in absence of pepsin (Figure 8B) and slight relaxed surface of beads in presence of pepsin due to peptic activity (Figure 8C). The hydrogen ion dissociation constant (pKa) of alginate is between 3.20-3.38 (Bu et al., 2005; Deng et al., 2010). At the pH below pKa, most of the carboxylic groups in the alginate exist in the form of -COOH. When SA micro-beads were incubated in SGF at pH 3.0 (pH close to pKa), some of carboxylic acid groups in alginate may exist as ionized form ($-COO^-$), as a result charge repulsion occurred between ionized carboxylate ($-COO^-$) groups, favouring matrix swelling. On the other hand, incubation of MP/SA micro-beads in SGF at pH 3.0 (pH < pI of MP 4.6-5.2; Doherty et al., 2011), polypeptide chains of MP were positively charged and some of carboxylate groups in alginate were in the form of $-COO^-$, therefore some ionic interaction may have occurred between positively charged polypeptide chains and negatively charged carboxylate groups, which favouring for matrix shrinkage. Another possible reason for shrinking of MP/SA gel micro-beads in gastric incubation, dense matrix lattice generated compact micro-beads which may not allow penetration of acidic media into the beads, possibly explaining the shrinkage of micro-beads and reduction of beads diameter.

Incubation of microbeads in SIF at pH 7.0 after following 120 min incubation in SGF, diameter of SA beads increased (25%) significantly without any degradation on 180 min incubation in absence of pancreatin (Figure 7B). On the other hand, a rapid and significant increase in diameter (42%) of MP/SA beads was detected within first 30 min in absence of pancreatin (Figure 7B). Beads diameter was further increased (up to 46%) on following 180 min incubation but no micro-beads degradation was observed. However, micro-beads were completely degraded within 30 min of incubation in presence of pancreatin and bile extract. Microscopic images of swelled MP/SA micro-beads in SIF in absence of pancreatin and degraded MP/SA micro-beads in presence of pancreatin are shown in Figure 8D and Figure 8E, respectively. In intestinal fluid incubation, SA and MP/SA micro-beads began to swell presumably due to an increase in the electrostatic repulsive forces at a pH above the pKa of alginate and above the pI of milk protein in MP/SA micro-beads. At intestinal pH (pH > pKa), the carboxylic acid groups in alginate were ionized and became -COO$^-$ form. Thus, the weakened H-bonding interaction between polymer chains and electrostatic repulsion from - COO$^-$ groups resulted in the higher swelling of alginate beads (Deng et al., 2010). Electrostatic repulsion was occurred in milk protein-based micro-beads during intestinal incubation because of pH increased to 7.0 or above (pH > p*I*), resulting net negative charges on protein particles and higher swelling rate (Doherty et al., 2011; Hébrard et al., 2013).

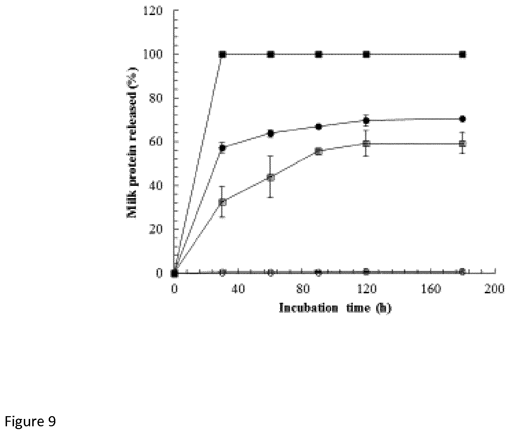

In SGF at pH 3.0, there was practically no protein release (< 0.6%) during incubation of MP/SA micro-beads for a 180min period in absence of pepsin. Chromatography analysis also confirmed no release of protein in gastric incubation in absence of pepsin (Figure 9). However, burst release of protein was detected in presence of pepsin due to proteolytic activity of enzyme (Figure 9). Most of the protein was released during the initial 30 min of incubation and then release pattern became slow. There was no significant difference in release of protein between 120 min and 180 min. Light microscopic observations showed that MP/SA micro-beads appeared intact after 180 min of gastric incubation (Figure 8C for 120 min of incubation). Due to shrinking of MP/SA micro-beads in acidic SGF the pore size of beads on surface may be decreased thus limiting the penetration of acidic media and no release of protein. On the other hand, protein digestion by pepsin was dominated on the surface of beads during initial incubation in presence of pepsin. But there was still limiting the penetration of macromolecular pepsin into the beads due to their contractile surface in gastric incubation and slow down protein released from interior.

In SIF at pH 7.0, a substantial release of protein was detected during initial 30 min of incubation along with a rapid swelling of micro-beads in absence of intestinal enzymes (Figure 9). Release of proteins was then gradually increased with increase of incubation time for up to 120 min and then followed by a plateau without degradation of micro-beads. On the other hand, microbeads were mostly degraded (97% protein release) within 30 min of incubation according to expectations in SIF in presence of pancreatin due to proteolytic activity of enzymes. Light microscopy validated no degradation of beads during intestinal incubation in absence of pancreatin (Figure 8D) and degradation of beads in presence of pancreatin (Figure 8E). Swelling of microbeads by electrostatic repulsion of proteins molecules at pH 7.0 may facilitate the penetration of pancreatin and thus accelerating the proteolysis for microbeads degradation.

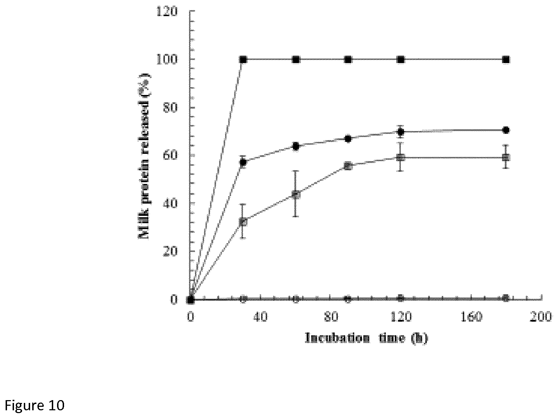

The release of folic acid from SA and MP/SA (65/35) micro-beads during incubation in SGF at pH 3.0 and in SIF at pH 7.0 in absence and presence of digestive enzymes are shown in Figure 10. In SGF, a small amount of folic acid was released from both SA (7.9%) and MP/SA (4.8%) micro-beads in absence of pepsin. In contrast, burst release of folic acid was detected in presence of pepsin. The release of folic acid from SA microbeads was relatively faster rather than from MP/SA micro-beads in both absence and presence of pepsin. However, most of the folic acid was released within the first 30 min of incubation followed by a nearly plateau state on subsequent incubation. The initial fast release can be explained as fast diffusion of water soluble folic acid from the surface of the micro-beads. Data also showed that the release behaviour of folic acid from micro-beads was related with swelling behaviour of matrices in simulated gastric-intestinal pH. Since encapsulation of folic acid in polymer gel matrices mainly occurred by physical entrapment (Deng et al., 2010), therefore swelling of SA microbeads in SGF at pH 3.0 may expand the surface pores of the beads, as a result folic acid can diffuse through these pores and release relatively faster rate from SA micro-beads. On the other hand, shrinking of MP/SA micro-beads may shrink the surface pores, resulting little chances to diffuse folic acid from inside the beads and reduced release rate. In presence of pepsin, some of polypeptide chains of proteins molecules were hydrolysed due to proteolytic activity of pepsin, as a result porous gel structure was formed (Figure 8C), which facilitated the diffusion of folic acid easily and released faster.

The incubation of microbeads in SIF at pH 7.0 after 120 min incubation in SGF, the release of folic acid was accelerated very fast with a release of about 83% from SA micro-beads and 68% from MP/SA microbeads within first 30 min due to fast swelling of beads. Subsequently the release rate became slow with a total release of 84% and 74% from SA and MP/SA microbeads, respectively after 180 min of incubation in absence of pancreatin without degradation of microbeads. In contrast, microbeads were degraded within 30 min of incubation with complete release of folic acid from both SA and MP/SA micro-beads when beads were incubated in SIF in presence of pancreatin. Fast and higher swelling of SA beads (Figure 7) than of MP/SA beads probably the reason for higher release of folic acid from SA beads in absence of enzymes.

Assessment of microbead formulation for its suitability as a delivery system for probiotic cultures.

There was a reduction in cell viability for the wet beads over the 7 days, i.e. $3.3 \times 10^{10}$ CFU/g and after 7 days $2.01 \times 10^{10}$ CFU/g. Even though a small reduction was seen, the cell viability in the beads was still high. These results show that inclusion of high amounts of bacteria did not negatively affect the bead formation, structure or storage stability of the microbeads.

**Conclusion**

Microscopy observation confirmed the microbeads integrity after 180 min incubation in SGF in presence of pepsin and disintegration within 30 min of incubation in SIF in presence of pancreatin. MP/SA microbeads delayed folic acid release in SGF and completely released in SIF in presence of enzymes. The microbeads of the current invention can protect encapsulated folic acid or other bioactive components from harsh gastric environmental condition and control release of the encapsulated component during gastric-intestinal passage to deliver them at specific target site.

**Claims**

1. A method for producing beads comprising an active component encased therein, the method comprising

   - providing a solution comprising milk protein product in which the milk protein has a degree of denaturation of at least 70%, alginate and an active component,
   - forming droplets with said solution; and
   - gelation of said droplets in a bath of a solution comprising a cation,

   to form beads.

2. The method of Claim 1, in which the milk protein has a degree of denaturation of between 80% and 100%.

3. The method of Claim 1 or 2, in which the milk protein product is heat-treated.

4. The method of Claim any one of the preceding Claims, in which the beads are microbeads or macrobeads.

5. The method of any one of the preceding Claims, in which the milk protein product is milk protein isolate or milk protein concentrate, preferably in which the milk protein product comprises 80% or more milk protein.

6. The method of any one of the preceding Claims, in which the alginate is sodium alginate.

7. The method of any one of the preceding Claims, wherein the concentration of the milk protein product in the solution is from about 0.5% w/w to about 6% w/w, preferably, wherein the concentration is less than about 3% w/w, and/or wherein the concentration of alginate is from about 0.3% w/w to about 3% w/w, preferably, in which the concentration of the active component is greater than 0 to about 4% w/w.

8. The method of any one of the preceding Claims, wherein the microbead is produced by extrusion of the mixture from a syringe through a vibrating nozzle, preferably in which the nozzle comprises an aperture of about 200 $\mu$m.

9. The method of any one of the preceding Claim, in which the cation is selected from the group comprising calcium, magnesium and iron, preferably in which the solution comprises calcium chloride.

10. The method of any one of the preceding Claims, in which the mixture is filtered prior to the steps of forming the droplets and gelation.

11. The method of any one of the preceding Claims wherein the active component is selected from the group comprising a probiotic, an antibody, an enzyme, a vitamin, a microorganism, a protein, a sugar, a peptide, a nucleic acid or nucleic acid construct and a pharmaceutically active agent.

12. A microbead preparation or macrobead preparation obtainable by the method of any one of Claims 1 to 11.

13. A microbead preparation comprising a mixture of milk protein product in which the milk protein has a degree of denaturation of at least 70%, and alginate, wherein the size of the microbead in the preparation is from about 150 $\mu$m to about 500 $\mu$m.

14. A delivery vehicle for delivery or transport of an active component to a site in the body, comprising a bead obtainable by the method of any one of Claims 1 to 11, or the bead preparation of Claim 12 or 13.

15. A food product or beverage product comprising a bead obtainable by the method of any one of Claims 1 to 11, or the bead preparation of Claim 12 or 13.

**Patentansprüche**

1. Verfahren für die Herstellung von Kügelchen, die eine Wirkkomponente umfassen, die darin eingeschlossen ist, wobei das Verfahren Folgendes umfasst:

   - Bereitstellen einer Lösung, die Folgendes umfasst: Milchproteinprodukt, in dem das Milchprotein einen Denaturierungsgrad von mindestens 70 % aufweist, Alginat und eine Wirkkomponente,
   - Bilden von Tröpfchen mit der Lösung; und
   - Gelierung der Tröpfchen in einem Bad einer Lösung, die ein Kation umfasst, um Kügelchen zu bilden.

2. Verfahren nach Anspruch 1, in dem das Milchprotein einen Denaturierungsgrad von zwischen 80 % und 100 % aufweist.

3. Verfahren nach Anspruch 1 oder 2, in dem das Milchproteinprodukt wärmebehandelt ist.

4. Verfahren nach einem der vorstehenden Ansprüche, in dem die Kügelchen Mikrokügelchen oder Makrokügelchen sind.

5. Verfahren nach einem der vorstehenden Ansprüche, in dem das Milchproteinprodukt Milchproteinisolat oder Milchproteinkonzentrat ist, bevorzugt in dem das Milchproteinprodukt 80 % oder mehr Milchprotein umfasst.

6. Verfahren nach einem der vorstehenden Ansprüche, in dem das Alginat Natriumalginat ist.

7. Verfahren nach einem der vorstehenden Ansprüche, wobei die Konzentration des Milchproteinprodukts in der Lösung von etwa 0,5 % (m/m) bis etwa 6 % (m/m) beträgt, bevorzugt wobei die Konzentration weniger als etwa 3 % (m/m) beträgt und/oder wobei die Konzentration von Alginat von etwa 0,3 % (m/m) bis etwa 3 % (m/m) beträgt, bevorzugt in dem die Konzentration der Wirkkomponente mehr als 0 bis etwa 4 % (m/m) beträgt.

8. Verfahren nach einem der vorstehenden Ansprüche, wobei das Mikrokügelchen durch Extrusion der Mischung aus einer Spritze durch eine vibrierende Düse hergestellt wird, bevorzugt in dem die Düse eine Öffnung von etwa 200 $\mu$m umfasst.

9. Verfahren nach einem der vorstehenden Ansprüche, in dem das Kation aus der Gruppe ausgewählt ist, die Calcium, Magnesium und Eisen umfasst, bevorzugt in dem die Lösung Calciumchlorid umfasst.

10. Verfahren nach einem der vorstehenden Ansprüche, in dem die Mischung vor den Schritten des Bildens der Tröpfchen und der Gelierung gefiltert wird.

11. Verfahren nach einem der vorstehenden Ansprüche, wobei die Wirkkomponente aus der Gruppe ausgewählt ist, die Folgende umfasst: ein Probiotikum, einen Antikörper, ein Enzym, ein Vitamin, einen Mikroorganismus, ein Protein, einen Zucker, ein Peptid, eine Nukleinsäure oder ein Nukleinsäurekonstrukt und einen pharmazeutischen Wirkstoff.

12. Mikrokügelchenpräparat oder Makrokügelchenpräparat, das durch das Verfahren nach einem der Ansprüche 1 bis 11 erhalten werden kann.

13. Mikrokügelchenpräparat, das eine Mischung aus Milchproteinprodukt, in dem das Milchprotein einen Denaturierungsgrad von mindestens 70 % aufweist, und Alginat umfasst, wobei die Größe des Mikrokügelchens in dem Präparat von etwa 150 $\mu$m bis etwa 500 $\mu$m beträgt.

14. Abgabevehikel für die Abgabe oder den Transport einer Wirkkomponente an/zu einem Ort in dem Körper, das ein Kügelchen, das durch das Verfahren nach einem der Ansprüche 1 bis 11 erhalten werden kann, oder das Kügelchenpräparat nach Anspruch 12 oder 13 umfasst.

15. Nahrungsmittelprodukt oder Getränkeprodukt, das ein Kügelchen, das durch das Verfahren nach einem der Ansprüche 1 bis 11 erhalten werden kann, oder das Kügelchenpräparat nach Anspruch 12 oder 13 umfasst.


**Revendications**

1. Méthode de production de billes comprenant un composant actif en étant enrobé, la méthode comprenant :

   - la mise à disposition d'une solution comprenant un produit de protéines du lait dans lequel les protéines du lait présentent un degré de dénaturation d'au moins 70%, de l'alginate et un composant actif ;
   - la formation de gouttelettes à partir de ladite solution ; et
   - la gélification desdites gouttelettes dans un bain d'une solution comprenant un cation ;

   afin de former des billes.

2. Méthode selon la revendication 1, dans laquelle les protéines du lait présentent un degré de dénaturation compris entre 80% et 100%.

3. Méthode selon la revendication 1 ou 2, dans laquelle le produit de protéines du lait est traité thermiquement.

4. Méthode selon l'une quelconque des revendications précédentes, dans laquelle les billes sont des microbilles ou des macrobilles.

5. Méthode selon l'une quelconque des revendications précédentes, dans laquelle le produit de protéines du lait est un isolat de protéines du lait ou un concentré de protéines du lait, préférablement où le produit de protéines du lait comprend 80%, ou plus, de protéines du lait.

6. Méthode selon l'une quelconque des revendications précédentes, dans laquelle l'alginate est l'alginate de sodium.

7. Méthode selon l'une quelconque des revendications précédentes, où la concentration en produit de protéines du lait dans la solution va d'environ 0,5% p/p à environ 6% p/p, préférablement, où la concentration est inférieure à environ 3% p/p, et/ou

   où la concentration en alginate va d'environ 0,3% p/p à environ 3% p/p, préférablement,
   où la concentration en composant actif est supérieure à 0 et va jusqu'à environ 4% p/p.

8. Méthode selon l'une quelconque des revendications précédentes, dans laquelle la microbille est produite par l'extrusion du mélange à partir d'une seringue dans une buse vibrante, préférablement où la buse présente une ouverture d'environ 200 $\mu$m.

9. Méthode selon l'une quelconque des revendications précédentes, dans laquelle le cation est choisi dans le groupe constitué par le calcium, le magnésium et le fer, préférablement où la solution comprend du chlorure de calcium.

10. Méthode selon l'une quelconque des revendications précédentes, dans laquelle le mélange est filtré préalablement aux étapes de formation des gouttelettes et de gélification.

11. Méthode selon l'une quelconque des revendications précédentes, dans laquelle le composant actif est choisi dans le groupe constitué par un probiotique, un anticorps, une enzyme, une vitamine, un microorganisme, une protéine, un sucre, un peptide, un acide nucléique, ou une construction d'acide nucléique, et un agent pharmaceutiquement actif.

**12.** Préparation de microbilles ou préparation de macrobilles, pouvant être obtenue par la méthode selon l'une quelconque des revendications 1 à 11.

**13.** Préparation de microbilles comprenant un mélange d'un produit de protéines du lait dans lequel les protéines du lait présentent un degré de dénaturation d'au moins 70%, et d'alginate, où la taille des microbilles dans la préparation va d'environ 150 $\mu$m à environ 500 $\mu$m.

**14.** Véhicule d'administration destiné à l'administration ou au transport d'un composant actif vers un site dans l'organisme, comprenant une bille pouvant être obtenue par la méthode selon l'une quelconque des revendications 1 à 11, ou la préparation de billes selon la revendication 12 ou 13.

**15.** Produit alimentaire ou produit de boisson, comprenant une bille pouvant être obtenue par la méthode selon l'une quelconque des revendications 1 à 11, ou la préparation de billes selon la revendication 12 ou 13.

Figure 1

(A) 3.1% MP/2.0% SA (75/25)

(B) 3.1% MP/2.0% SA (70/30)

(C) 3.1% MP/2.0% SA (65/35)

(D) 3.1% MP/2.0% SA (60/40)

(E) 3.1% MP/2.0% SA (0/100)

Figure 2

Figure 3

3.1% MP/2.0% SA (75/25)    3.1% MP/2.0% SA (70/30)

3.1% MPI/2.0% SA (65/35)    3.1% MPI/2.0% SA (60/40)

Figure 4

Figure 5

Figure 6

Figure 7

Figure 8

EP 3 554 479 B1

Figure 9

28

Figure 10

Figure 11

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02094224 A

**Non-patent literature cited in the description**

- **DOHERTY et al.** *Food Hydrocolloids,* 2011, vol. 25, 1604-1617